# EUROPÄISCHE PATENTANMELDUNG

(11) **EP 2 505 155 A1**
(43) Veröffentlichungstag der Anmeldung: **03.10.2012**
(21) Anmeldenummer: 11160709.9
(22) Anmeldetag: 31.03.2011
(51) Int. Cl.: A61B 17/70

(54) **Wirbelsäulenimplantat zur Stabilisierung und Versteifung von Wirbelkörpern einer Wirbelsäule**

(71) Anmelder: Spinelab AG, 8406 Winterthur (CH)
(72) Erfinder: Clark, Jonathan, 8302, Kloten (CH); Braunschweiler, Reto, 8413, Neftenbach (CH); Zehnder, Thomas, 8806, Bäch (CH)
(74) Vertreter: BOVARD AG

(57) **Zusammenfassung**

Ein Wirbelsäulenimplantat zur Stabilisierung und Versteifung von Wirbelkörpern einer Wirbelsäule umfasst mindestens eine erste Knochenschraube (3), welche in einen Wirbelkörper einschraubbar ist, und mindestens eine zweite Knochenschraube (10), welche ebenfalls in einen Wirbelkörper einschraubbar. Ein erstes Verbindungselement (2), das starr ist, ist in erste Aufnahmemittel (5) der ersten Knochenschraube (3) einsetzbar. Ein zweites Verbindungselement (9), das elastisch ist, ist in zweite Aufnahmemittel (13) der zweiten Knochenschraube einsetzbar. Über Verbindungsmittel (16) wird das erste Verbindungselement (2) mit dem zweiten Verbindungselement (9) verbunden. Die Verbindungsmittel (16) umfassen zwei Schenkel (20), (21), die eine U-förmige Ausnehmung (22) bilden und an einer weiteren Knochenschraube (18) angebracht sind. In einem ersten Bereich der U-förmigen Ausnehmung (22) ist ein Endbereich eines ersten Verbindungselementes (2) einsetzbar, in einem zweiten Bereich der U-förmigen Ausnehmung (22) ist ein Endbereich eines zweiten Verbindungselementes (9) einsetzbar. Zwischen die beiden Schenkel (20) und (21) ist ein Presselement (25) eingesetzt, auf die Schenkel (20) und (21) sind Spannmittel (28) aufgesetzt, mit welchen das Presselement (25) gegen die beiden Endbereiche des ersten und zweiten Verbindungselementes (2) bzw. (9) gepresst wird.

## Beschreibung

Die vorliegende Erfindung bezieht sich auf ein Wirbelsäulenimplantat zur Stabilisierung und Versteifung von Wirbelkörpern einer Wirbelsäule, umfassend mindestens eine erste Knochenschraube, bestehend aus einem Einschraubteil, welcher in einen Wirbelkörper einschraubbar ist, und ersten Aufnahmemitteln, mindestens eine zweite Knochenschraube, bestehend aus einem Einschraubteil, welcher in einen Wirbelkörper einschraubbar ist, und zweiten Aufnahmemitteln, mindestens ein erstes Verbindungselement, das starr ist und in die ersten Aufnahmemittel der ersten Knochenschraube einsetzbar und darin befestigbar ist, mindestens ein zweites Verbindungselement, das elastisch ist und in die zweiten Aufnahmemittel der zweiten Knochenschraube einsetzbar und darin befestigbar sind, und Verbindungsmittel, mit welchen jeweils ein erstes Verbindungselement mit einem zweiten Verbindungselement verbindbar ist.

Derartige Wirbelsäulenimplantate zur Stabilisierung und Versteifung von Wirbelkörpern einer Wirbelsäule sind in vielfältiger Weise bekannt. Mit dem versteifenden Teil dieses Wirbelsäulenimplantats wird erreicht, dass zwischen den derartig versteiften Wirbelkörpern ein knöchernes Verwachsen stattfindet, was zu einer gewünschten Stabilisierung der Wirbelsäule führt, während mit dem elastischen Bereich des Wirbelsäulenimplantats die Wirbelkörper stützend stabilisiert werden, eine gewisse Beweglichkeit zwischen den einzelnen Wirbelkörpern ist erwünscht und wird zugelassen.

Ein derartiges Wirbelsäulenimplantat zur Stabilisierung und Versteifung von Wirbelkörpern einer Wirbelsäule ist beispielsweise aus der EP- A-1 961 392 bekannt.

Bei diesen bekannten Wirbelsäulenimplantaten muss das Setzen der Knochenschrauben in den einzelnen Wirbelkörpern insbesondere im Bereich des Übergangs zum starren Verbindungselement sehr genau erfolgen. Gewisse Ungenauigkeiten können dadurch auskorrigiert werden, indem das starre Verbindungselement passend gebogen wird, was während des Einsetzens des Wirbelsäulenimplantats in eine Wirbelsäule ausgeführt werden muss und den Eingriff erschwert. Zudem ist das Verbinden des starren Verbindungselementes mit dem elastischen Verbindungselement aufwändig, da jedes einzelne Verbindungselement separat in das jeweilige Verbindungsmittel eingesetzt und über eine eigene Spanneinrichtung fixiert wird, was einen relativ komplizierten Aufbau des entsprechenden Verbindungsmittels erfordert und was auch den Eingriff zusätzlich erschwert.

Die Aufgabe der vorliegenden Erfindung besteht somit darin, ein Wirbelsäulenimplantat zur Stabilisierung und Versteifung von Wirbelkörpern einer Wirbelsäule zu schaffen, das einfach im Aufbau ist, wodurch insbesondere das Einsetzen und Verbinden der starren und elastischen Verbindungselemente vereinfacht wird und mit welchem eine optimale Verbindung der starren Verbindungselemente und der elastischen Verbindungselemente erreicht werden kann.

Erfindungsgemäss erfolgt die Lösung dieser Aufgabe dadurch, dass die Verbindungsmittel zwei Schenkel umfassen, die eine U-förmige Ausnehmung bilden und an einer weiteren Knochenschraube angebracht sind, dass in einen ersten Bereich der U-förmigen Ausnehmung ein Endbereich eines ersten Verbindungselementes einsetzbar ist und in einen zweiten Bereich der U-förmigen Ausnehmung ein Endbereich eines zweiten Verbindungselementes einsetzbar ist, dass zwischen die beiden Schenkel ein Presselement einsetzbar und auf die Schenkel Spannmittel aufsetzbar sind, mit welchem das Presselement gegen die beiden Endbereiche des ersten und zweiten Verbindungselementes pressbar ist.

Mit dieser erfindungsgemässen Ausgestaltung wird erreicht, dass mit einem einzigen Spannvorgang sowohl das starre Verbindungselement als auch das elastische Verbindungselement in der U-förmigen Ausnehmung der weiteren Knochenschraube eingespannt und fixiert werden können, wodurch die Handhabung massiv vereinfacht wird.

In vorteilhafter Weise ist das Presselement mit Führungen ausgestattet, welche mit an den Flanken der Schenkel angeordneten Führungsflächen zusammenwirken, wodurch das Presselement geführt auf die Schenkel aufgesetzt werden kann, was diesen Vorgang vereinfacht.

In vorteilhafter Weise umfassen die Spannmittel eine Spannschraube, mit welcher das Presselement mit einer ersten Pressfläche gegen den Endbereich des ersten Verbindungselementes und einer zweiten Pressfläche gegen den Endbereich des zweiten Verbindungselementes pressbar ist, was einen einfachen Aufbau und einen einfachen Spannvorgang ergibt.

In vorteilhafter Weise ist die Spannschraube in eine Kappe eingesetzt ist, die auf die Schenkel aufsetzbar und mit diesen verriegelbar sind, wodurch ein einfaches Einsetzen der Spannmittel in die Knochenschraube erreicht wird.

Dies kann auch dadurch erreicht werden, indem die Spannschraube in ein innenseitig an den Schenkeln angebrachtes Gewinde einschraubbar ist.

In vorteilhafter Weise sind die Spannmittel aus einer Kappe gebildet, die auf die Schenkel aufsetzbar und mit diesen verriegelbar sind, und ist in die Kappe eine Spannschraube eingesetzt, mit welcher das Presselement mit einer ersten Pressfläche gegen den Endbereich des ersten Verbindungselementes und einer zweiten Pressfläche gegen den Endbereich des zweiten Verbindungselementes pressbar ist, was einem einfachen Aufbau dieser Spannmittel und dadurch eine einfache Handhabung ergibt.

Eine weitere vorteilhafte Ausgestaltung der Erfindung besteht darin, dass der Endbereich des ersten Verbindungselementes kugelförmig ausgebildet ist und dass die zwischen dem ersten Schenkel und dem zweiten Schenkel gebildete Grundfläche des ersten Bereichs der U-förmigen Ausnehmung eine konkave Kugelform aufweist, die dem kugelförmigen Endbereich des ersten Verbindungselementes entspricht. Dadurch wird erreicht, dass das erste Verbindungselement, das starr ist, bezüglich der Spannmittel und somit dem zweiten elastischen Verbindungselement allseitig in einem gewissen Winkelbereich ausgerichtet werden kann und in optimaler Weise an die in die Wirbelkörper eingesetzten Knochenschrauben angepasst werden kann, wodurch erreicht wird, dass ein nicht exaktes Setzen der Knochenschrauben in die Wirbelkörper statt durch aufwändiges Verbiegen des starren Verbindungselementes während des Eingriffs durch die Möglichkeit einer polyaxialen Ausrichtung des ersten Verbindungselementes ausgeglichen werden kann.

Um eine optimale Spannwirkung und demzufolge optimale Fixierung der Verbindungselemente zu erhalten, sind die jeweiligen Pressflächen des Presselementes an die Oberflächenformen der jeweiligen Endbereiche der Verbindungselemente angepasst.

In vorteilhafter Weise sind die zweite Pressfläche des Presselementes und/oder die Grundfläche des zweiten Bereichs der U-förmigen Ausnehmung mit Strukturen versehen, die in die Oberfläche des elastischen Verbindungselementes eindringen können und dadurch eine optimale Verbindung erhalten wird.

In vorteilhafter Weise ist mindestens der Endbereich des zweiten Verbindungselementes mit Strukturen versehen, die zu den Strukturen des zweiten Bereichs der U-förmigen Ausnehmung komplementär ausgebildet sind, wodurch eine formschlüssige Verbindung erhalten wird.

Eine derartige optimale formschlüssige Verbindung wird dann erreicht, wenn die Strukturen aus Rippen und Rillen bestehen, die im Wesentlichen quer zur Längsachse des zweiten Verbindungselementes ausgerichtet sind.

In vorteilhafter Weise ist die Spannschraube drehbar mit dem Presselement verbunden, was ermöglicht, die Kappe, die darin eingeschraubte Spannschraube und das Presselement als Einheit auf die weitere Knochenschraube aufzusetzen, wodurch die Handhabung massiv vereinfacht wird.

Ein weitere vorteilhafte Ausgestaltung der Erfindung besteht darin, dass das Presselement mindestens auf der Seite, welche mit dem zweiten Verbindungselement zusammenwirkt, mit Abstützflächen versehen ist, welche auf an den Schenkeln angebrachten Anschlagflächen abstützbar sind. Dadurch wird erreicht, dass der eingespannte Endbereich des elastischen zweiten Verbindungselementes nicht einer zu starken Pressung unterworfen wird.

Eine Ausgestaltung der Erfindung wird nachfolgend anhand der beiliegenden Zeichnungen beispielhaft näher erläutert.

Es zeigt
Fig. 1 in räumlicher Darstellung ein Wirbelsäulenimplantat, das ein erstes starres Verbindungselement, ein zweites elastisches Verbindungselement und Knochenschrauben, in welche die Verbindungselemente eingesetzt und gehalten sind und eine weitere Knochenschraube umfasst, in welcher die Endbereiche der zwei Verbindungselemente eingesetzt und fixiert sind:
Fig. 2 in räumlicher Darstellung die Ausführungsform gemäss Fig. 1, wobei die Einzelteile der Spannmittel der weiteren Knochenschraube in auseinandergezogenem Zustand dargestellt sind;
Fig. 3 eine vergrösserte Darstellung der Aufnahme für die Verbindungselemente der weiteren Knochenschraube gemäss Fig. 2;
Fig. 4 eine Längsschnittdarstellung des Wirbelsäulenimplantates gemäss Fig. 1 und Fig. 2;
Fig. 5 in räumlicher Darstellung eine weitere Ausführungsform eines erfindungsgemässen Wirbelsäulenimplantats;
Fig. 6 in räumlicher Darstellung die Ausführungsform gemäss Fig. 5, wobei die Einzelteile der Spannmittel der weiteren Knochenschraube in auseinandergezogenem Zustand dargestellt sind; und
Fig. 7 eine Längsschnittdarstellung des Wirbelsäulenimplantates gemäss Fig. 5 und Fig. 6.

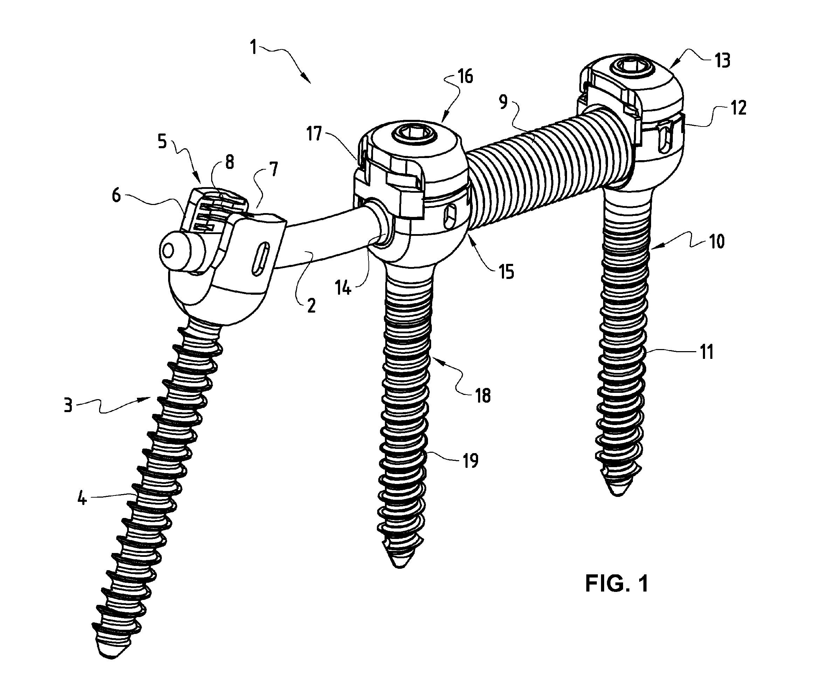

Fig. 1 zeigt in räumlicher Darstellung ein Wirbelsäulenimplantat 1 das ein erstes Verbindungselement 2 aufweist, das starr ist. Dieses erste Verbindungselement 2 ist in einer ersten Knochenschraube 3 gehalten, die in bekannter Weise aus einem Einschraubteil 4 besteht, welcher ebenfalls in bekannter, nicht dargestellter Weise in einen Wirbelkörper eingeschraubt werden kann. Zur Halterung des ersten Verbindungselementes 2 ist diese erste Knochenschraube 3 mit ersten Aufnahmemitteln 5 ausgestattet, die im Kopfteil 6 der ersten Knochenschraube 3 als U-förmige Aufnahme 7 ausgebildet sind. Das erste Verbindungselement 2, das als Stab ausgebildet ist, kann in diese U-förmige Aufnahme 7 eingelegt werden, die U-förmige Aufnahme 7 weist innenseitig ein Gewinde 8 auf, in welches in bekannter, nicht dargestellter Weise eine Spannschraube eingeschraubt werden kann, mittels welcher das erste Verbindungselement 2 in die U-förmige Aufnahme 7 eingespannt wird, wie später noch im Detail beschrieben wird.

Das hier dargestellte Wirbelsäulenimplantat 1 umfasst ferner ein zweites Verbindungselement 9, das elastisch ist und das in einer zweiten Knochenschraube 10 gehalten ist. Diese zweite Knochenschraube 10 besteht ebenfalls aus einem Einschraubteil 11, welcher in bekannter, nicht dargestellter Weise in einen Wirbelkörper eingeschraubt werden kann. Im Kopfteil 12 dieser zweiten Knochenschraube 10 sind zweite Aufnahmemittel 13 angeordnet, in welchen das zweite Verbindungselement 9 in der zweiten Knochenschraube 10 fixiert und gehalten werden kann. Eine derartige zweite Knochenschraube mit den zweiten Aufnahmemitteln für die Aufnahme des zweiten Verbindungselementes ist in der europäischen Patentanmeldung mit der Veröffentlichungsnummer 2 074 957, auf welche hiermit verwiesen wird, im Detail beschrieben.

Wie aus Fig. 1 ersichtlich ist, ist der Endbereich 14 des ersten Verbindungselementes und der Endbereich 15 des zweiten Verbindungselementes in Verbindungsmitteln 16 verbindend gehalten. Diese Verbindungsmittel 16 sind im Kopfteil 17 einer weiteren Knochenschraube 18 angeordnet, welche weitere Knochenschraube 18 ebenfalls mit einem Einschraubteil 19 ausgestattet ist, mit welchem diese in bekannter, nicht dargestellter Weise ebenfalls in einen Wirbelkörper eingeschraubt werden kann.

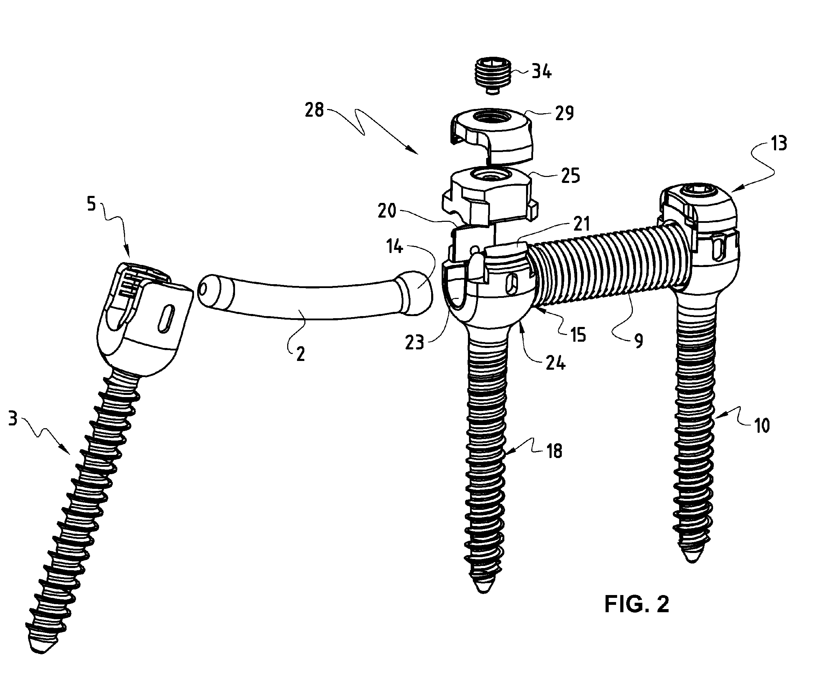

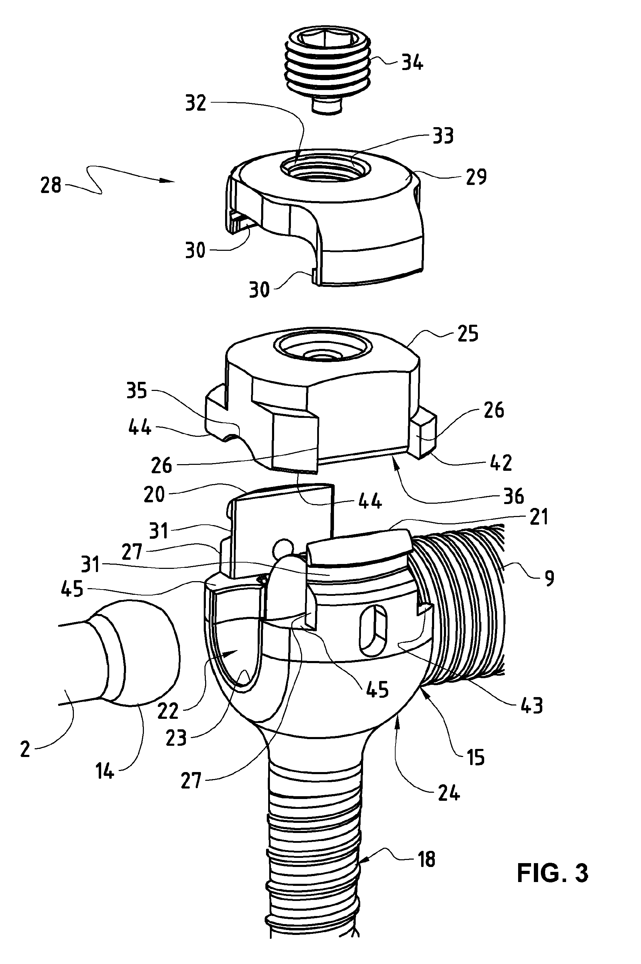

Wie aus Fig. 2 und insbesondere aus Fig. 3 ersichtlich ist, umfassen die Verbindungsmittel 16, die im Kopfteil 17 der weiteren Knochenschraube 18 angebracht sind, zwei Schenkel 20 und 21, die im Kopfteil 17 eine U-förmige Ausnehmung 22 bilden. In diese U-förmige Ausnehmung 22 ist in einem ersten Bereich 23 ein Endbereich 14 des ersten Verbindungselementes 2 einsetzbar. In einem zweiten Bereich 24 dieser U-förmigen Ausnehmung 22 ist ein Endbereich 15 des zweiten Verbindungselementes eingesetzt.

Zwischen die beiden Schenkel 20 und 21 ist ein Presselement 25 einsetzbar. Dieses Presselement 25 ist mit Führungen 26 in Form von Nocken ausgestattet. Diese Führungen 26 verlaufen beim Aufsetzen des Presselementes 25 auf die weitere Knochenschraube 18 entlang Führungsflächen 27, die jeweils an den Flanken der Schenkel 20 und 21 angebracht sind. Dadurch lässt sich das Presselement 25 geführt zwischen die beiden Schenkel 20 und 21 der weiteren Knochenschraube 18 einsetzen. Dieses Presselement 25 lässt sich mittels Spannmittel 28 gegen die Endbereiche 14 und 15 des ersten Verbindungselementes 2 bzw. des zweiten Verbindungselementes 9, wenn diese in die U-förmige Ausnehmung 22 eingesetzt sind, spannen. Diese Spannmittel 28 bestehen aus einer Kappe 29, welche auf die beiden Schenkel 20 und 21 aufgesetzt werden kann und mit diesen verriegelbar ist. Hierzu weist diese Kappe 29 zwei Rippen 30 auf, welche Rippen 30 sich in bekannter Weise in an der Aussenseite der Schenkel 20 und 21 angebrachte Nuten 31 eindrehen lassen. Im eingedrehten Zustand ist die Kappe 29 mit den beiden Schenkeln 20 und 21 verbunden.

Die Kappe 29 ist mit einer Bohrung 32 versehen, die mit einem Gewinde 33 ausgestattet ist. In das Gewinde 33 dieser Bohrung 32 ist eine Spannschraube 34 einschraubbar. Im montierten Zustand, wie dieser in Fig. 1 dargestellt ist, kann durch Anziehen dieser Spannschraube 34 das Presselement 25 gegen die Endbereiche 14 und 15 des ersten Verbindungselementes 2 bzw. des zweiten Verbindungselementes 9 gespannt werden, wobei das Presselement 25 mit einer ersten Pressfläche 35 gegen den Endbereich 14 des ersten Verbindungselementes 2 und einer zweiten Pressfläche 36 gegen den Endbereich 15 des zweiten Verbindungselementes 9 gepresst wird.

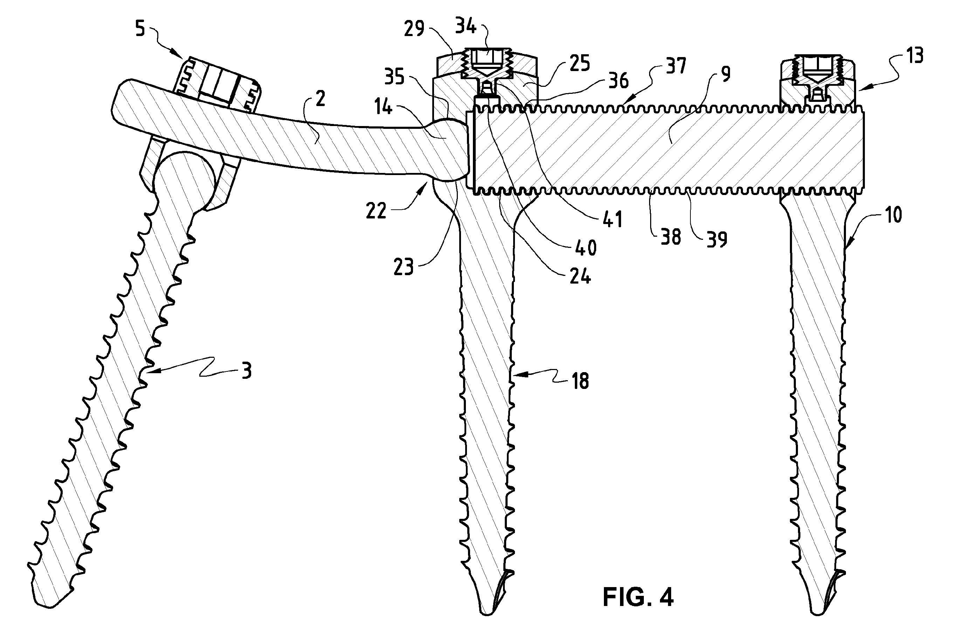

Beim in den Fig. 1 bis Fig. 4 dargestellten Ausführungsbeispiel ist der Endbereich 14 des ersten Verbindungselementes 2 kugelförmig ausgebildet. Die zwischen dem ersten Schenkel 20 und dem zweiten Schenkel 21 gebildete Grundfläche des ersten Bereiches 23 der U-förmigen Ausnehmung 22 weist eine konkave Kugelform auf, die dem kugelförmigen Endbereich 14 des ersten Verbindungselementes 2 entspricht, wie dies insbesondere aus Fig. 4 ersichtlich ist. Die erste Pressfläche 35 des Presselementes 25 weist ebenfalls eine konkave Kugelfläche auf, die der Kugelform des Endbereichs 14 des ersten Verbindungselementes 2 entspricht. Dadurch kann die Kugelform des Endbereichs 14 des ersten Verbindungselementes 2 in optimaler Weise in der weiteren Knochenschraube 18 gehalten und fixiert werden, es ist dadurch möglich, dass das erste Verbindungselement 2 bezüglich der weiteren Knochenschraube 18 und somit dem zweiten Verbindungselement 9 in einem gewissen Bereich winklig ausgerichtet werden kann, wobei trotzdem eine optimale Einspannung erreicht wird.

Wie ebenfalls aus Fig. 4 ersichtlich ist, ist das zweite Verbindungselement 9 an der Oberfläche mit Strukturen 37 versehen. Die zweite Pressfläche 36 des Presselementes 25 und die Grundfläche des zweiten Bereichs 24 der U-förmigen Ausnehmung 22 sind ebenfalls mit Strukturen versehen, die zu den Strukturen 37 des zweiten Verbindungselementes 9 komplementär ausgebildet sind. Dadurch erhält man in der weiteren Knochenschraube 18 mit dem zweiten Verbindungselement 9, das aus einem elastischen Material gebildet ist, eine formschlüssige Verbindung. Selbstverständlich wäre es auch denkbar, dass das zweite Verbindungselement 9 eine glatte Oberfläche aufweist, und dass nur die Grundfläche des zweiten Bereichs 24 der U-förmigen Ausnehmung 22 und/oder die zweite Pressfläche 36 des Presselementes 25 mit Strukturen versehen sind, die beim Spannen der Spannschraube 34 gegen das Presselement 25 hin in die Oberfläche des zweiten Verbindungselementes 9 eindringen würden, wodurch ebenfalls eine formschlüssige Verbindung entstehen würde.

Die Strukturen 37 könnten beispielsweise als Rippen 38 und Rillen 39 ausgebildet sein, die im Wesentlichen quer zur Längsachse des zweiten Verbindungselementes 9 ausgerichtet sein könnten, wodurch ein optimaler Formschluss entstehen würde.

Wie ebenfalls aus Fig. 4 ersichtlich ist, kann die Spannschraube 34 drehbar im Presselement 25 gehalten sein, wozu diese Spannschraube 34 mit einem Bolzen 40 ausgestattet ist, welcher durch eine im Presselement 25 angebrachte Bohrung 41 ragt, wobei der untere Teil des Bolzens 40 gekrempt wird, sodass die Spannschraube drehbar im Presselement 25 gehalten ist. Dadurch können die Spannmittel 28, bestehend aus Kappe 29 und Spannschraube 34 mit dem Presselement 25 vormontiert werden, diese können dann als Einheit auf die weitere Knochenschraube 18 aufgesetzt, verriegelt und entsprechend gespannt werden, was die Handhabung bei einem operativen Eingriff massiv erleichtert.

Wie aus Fig. 3 ersichtlich ist, weist das Presselement 25 auf der Seite, welche mit dem zweiten Verbindungselement 9 zusammenwirkt, Abstützflächen 42 auf. An den Schenkeln 20 und 21 sind entsprechende Anschlagflächen 43 angebracht. Die Abstützflächen 42 und die Anschlagflächen 43 sind so aufeinander ausgerichtet, dass bei voll gespanntem Zustand des Presselementes 25 und wenn eine genügende Presskraft auf das zweite Verbindungselement 9, das aus einem elastischen Material besteht, wirkt, sich Abstützflächen 42 auf den Anschlagflächen 43 abstützen, sodass eine zu starke Pressung des elastischen zweiten Verbindungselementes 9 im eingespannten Zustand vermieden wird.

Das Presselement 25 kann auch auf der Seite, welche mit dem ersten Verbindungselement 2 zusammenwirkt, mit Abstützflächen 44 versehen sein, welche auf an den Schenkeln 20 und 21 angebrachten Anschlagflächen 45 abstützbar sind. Die Abstützflächen 44 und die Anschlagflächen 45 können so aufeinander abgestimmt sein, dass bei eingesetztem ersten Verbindungselement 2 diese nicht in Kontakt kommen miteinander, es ist aber denkbar, dass das erste Verbindungselement, wenn die stabilisierten Wirbelkörper eine stabile Knochenverbindung aufgebaut haben, entfernt wird, beim Spannen des Presselements 25 nunmehr nur noch auf das zweite Verbindungselement 9 würden dann die Abstützflächen 44 auf den Anschlagflächen 45 abgestützt, es würde trotzdem eine stabile Spannung des zweiten Verbindungselementes 9 in der weiteren Knochenschraube 18 erreicht werden können.

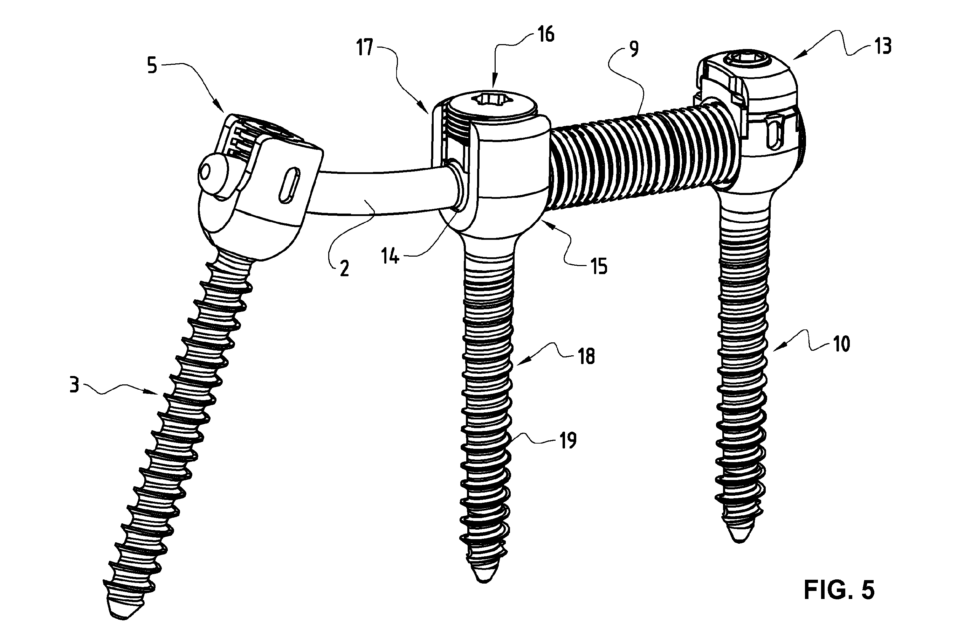

Die weitere Ausführungsform eines Wirbelsäulenimplantats, wie es aus Fig. 5 ersichtlich ist, besteht, wie das vorgängig beschriebene Beispiel, aus einem ersten starren Verbindungselement 2, das wiederum in einer ersten Knochenschraube 3 gehalten ist. Diese erste Knochenschraube 3 weist ebenfalls erste Aufnahmemittel 5 für die Aufnahme des ersten Verbindungselementes 2, das als Stab ausgebildet ist, auf.

Das elastische zweite Verbindungselement 9 ist in einer zweiten Knochenschraube 10 gehalten, wie dies zum vorgängig dargestellten Ausführungsbeispiel beschrieben worden ist.

Der Endbereich 14 des ersten Verbindungselementes 2 und der Endbereich 15 des zweiten Verbindungselementes 9 sind wiederum in Verbindungsmitteln 16 verbindend gehalten. Diese Verbindungsmittel 16 sind im Kopfteil 17 einer weiteren Knochenschraube 18 angeordnet, welche weitere Knochenschraube 18 ebenfalls mit einem Einschraubteil 19 ausgestattet ist, mit welchem diese in bekannter, nicht dargestellter Weise ebenfalls in einen Wirbelkörper eingeschraubt werden kann.

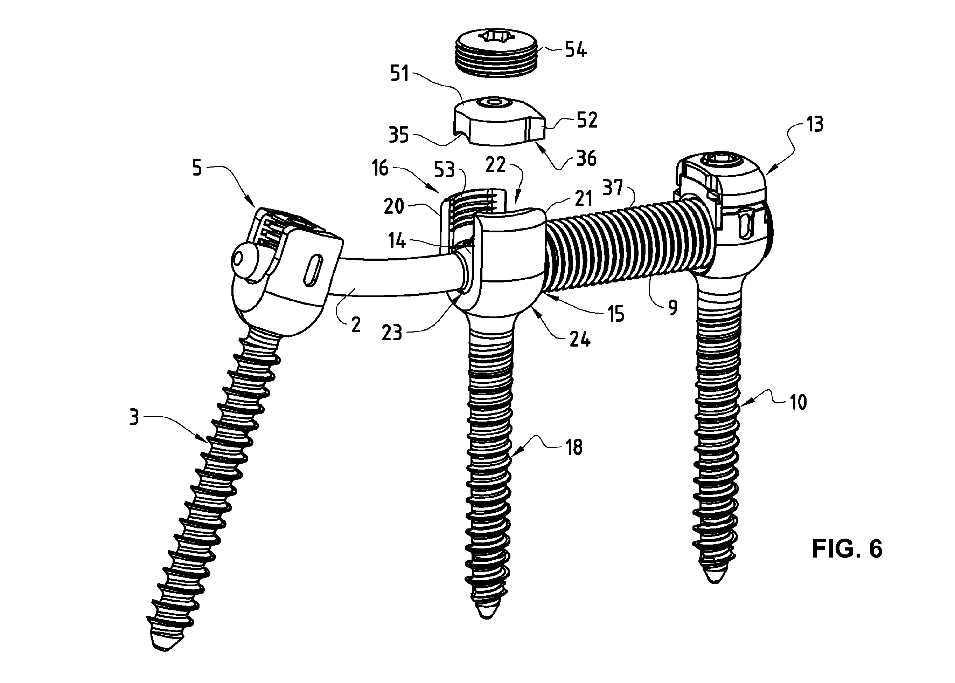

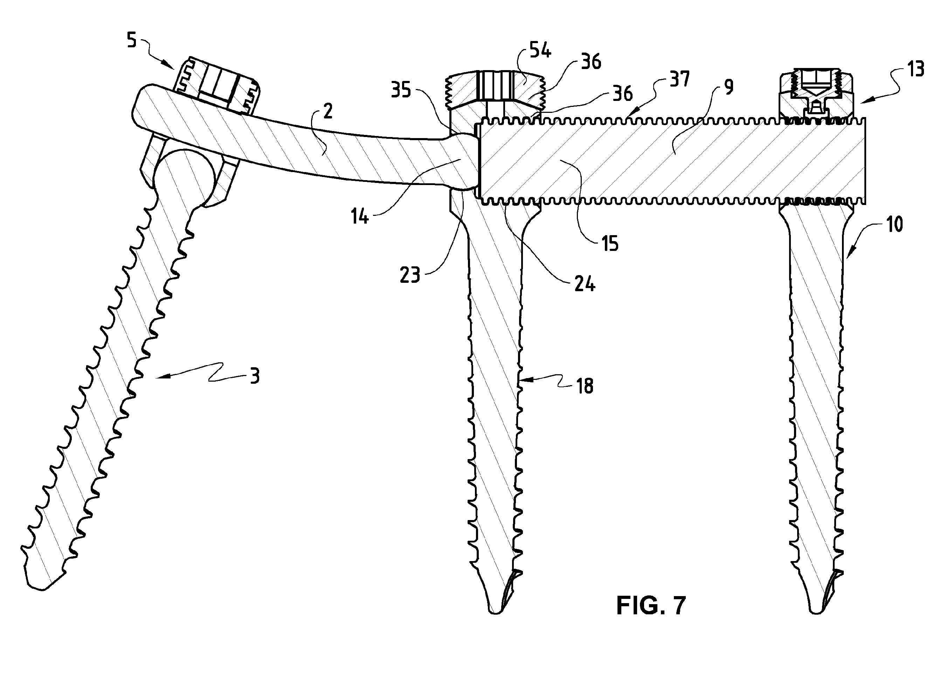

Wie aus Fig. 6 und aus Fig. 7 ersichtlich ist, umfassen die Verbindungsmittel 16, die im Kopfteil 17 der weiteren Knochenschraube 18 angebracht sind, wiederum zwei Schenkel 20 und 21, die im Kopfteil 17 eine U-förmige Ausnehmung 22 bilden. In diese U-förmige Ausnehmung 22 ist in einem ersten Bereich 23 ein Endbereich 14 des ersten Verbindungselementes 2 einsetzbar. In einem zweiten Bereich 24 dieser U-förmigen Ausnehmung 22 ist ein Endbereich 15 des zweiten Verbindungselementes eingesetzt.

Zwischen die beiden Schenkel 20 und 21 kann ein Presselement 51 eingesetzt werden. Dieses Presselement 51 ist mit einem Nocken 52 ausgestattet, der als Führung beim Einsetzen des Presselements 51 zwischen die beiden Schenkel 20 und 21 dient. Innenseitig an den beiden Schenkeln ist ein Gewinde 53 angebracht, in welches sich eine Spannschraube 54 einschrauben lässt. Durch Anziehen dieser Spannschraube 54 kann das Presselement 51 wiederum gegen die Endbereiche 14 und 15 des ersten Verbindungselementes 2 bzw. des zweiten Verbindungselementes 9 gespannt werden, das Presselement 51 wird hierbei wiederum mit einer ersten Pressfläche 35 gegen den Endbereich 14 des ersten Verbindungselementes 2 und einer zweiten Pressfläche 36 gegen den Endbereich 15 des zweiten Verbindungselementes 9 gepresst.

Auch beim hier dargestellten weiteren Ausführungsbeispiel ist der Endbereich 14 des ersten Verbindungselementes 2 kugelförmig ausgebildet. Die zwischen dem ersten Schenkel 20 und dem zweiten Schenkel 21 gebildete Grundfläche des ersten Bereiches 23 der U-förmigen Ausnehmung 22 weist eine konkave Kugelform auf, die dem kugelförmigen Endbereich 14 des ersten Verbindungselementes 2 entspricht, wie dies insbesondere aus Fig. 7 ersichtlich ist. Die erste Pressfläche 35 des Presselementes 25 weist ebenfalls eine konkave Kugelfläche auf, die der Kugelform des Endbereichs 14 des ersten Verbindungselementes 2 entspricht. Dadurch kann die Kugelform des Endbereichs 14 des ersten Verbindungselementes 2 in optimaler Weise in der weiteren Knochenschraube 18 gehalten und fixiert werden, es ist dadurch möglich, dass das erste Verbindungselement 2 bezüglich der weiteren Knochenschraube 18 und somit dem zweiten Verbindungselement 9 in einem gewissen Bereich winklig ausgerichtet werden kann, wobei trotzdem eine optimale Einspannung erreicht wird.

Entsprechend dem vorgängig beschriebenen Ausführungsbeispiel sind auch bei diesem weiteren Ausführungsbeispiel Strukturen 37 am zweiten Verbindungselement 9 vorgesehen, um eine formschlüssige Verbindung zu erhalten.

Das in derartigen Wirbelsäulenimplantaten eingesetzte elastische zweite Verbindungsmittel besteht in vorteilhafter Weise aus einem biokompatiblen Kunststoff auf Polyurethan-Basis, während die anderen Teile aus einer Titanlegierung bestehen. Selbstverständlich wären auch andere geeignete Materialien verwendbar.

Vorgängig sind Ausführungsbeispiele beschrieben worden, die jeweils insgesamt aus drei Schrauben gebildet sind, wobei die erste und die mittlere Schraube über ein starres Verbindungselement miteinander verbunden sind, und die mittlere Schraube und die zweite Schraube über ein elastisches Verbindungselement miteinander verbunden sind. Selbstverständlich sind auch viele andere Ausführungsbeispiele denkbar, die mehr Schrauben aufweisen, bei welchen die starren und elastischen Verbindungselemente sich beispielsweise über mehr als zwei Schrauben erstrecken, es ist auch denkbar, dass mehr als zwei Verbindungselementteile verwendet werden, beispielsweise kann zwischen zwei elastischen Verbindungselementen ein starres Verbindungselement eingesetzt werden, wobei im Bereich der Verbindungen dieser Verbindungselemente die vorgängig beschriebenen weiteren Knochenschrauben einzusetzen wären. Je nach Bedarf kann somit ein praktisch beliebig zusammengestelltes Wirbelsäulenimplantat verwendet werden.

Mit dieser Erfindung wird ein Wirbelsäulenimplantat zur Stabilisierung und Versteifung von Wirbelkörpern einer Wirbelsäule erhalten, die in optimaler Weise an die Begebenheiten angepasst werden kann, welche einfach ist in der Handhabung und mit welcher in ebenfalls optimaler Weise eine polyaxiale Anpassung eines starren Verbindungselementes bezüglich eines elastischen Verbindungselementes erreichbar ist.

## Patentansprüche

1. Wirbelsäulenimplantat zur Stabilisierung und Versteifung von Wirbelkörpern einer Wirbelsäule, umfassend mindestens eine erste Knochenschraube (3), bestehend aus einem Einschraubteil (4), welcher in einen Wirbelkörper einschraubbar ist, und ersten Aufnahmemitteln (5), mindestens eine zweite Knochenschraube (10), bestehend aus einem Einschraubteil (11), welcher in einen Wirbelkörper einschraubbar ist, und zweiten Aufnahmemitteln (13), mindestens ein erstes Verbindungselement (2), das starr ist und in die ersten Aufnahmemittel (5) der ersten Knochenschraube (3) einsetzbar und darin befestigbar ist, mindestens ein zweites Verbindungselement (9), das elastisch ist und in die zweiten Aufnahmemittel (13) der zweiten Knochenschraube (10) einsetzbar und darin befestigbar sind, und Verbindungsmittel (16), mit welchen jeweils ein erstes Verbindungselement (2) mit einem zweiten Verbindungselement (9) verbindbar ist, **dadurch gekennzeichnet, dass** die Verbindungsmittel (16) zwei Schenkel (20, 21) umfassen, die eine U-förmige Ausnehmung (22) bilden und an einer weiteren Knochenschraube (18) angebracht sind, dass in einen ersten Bereich (23) der U-förmigen Ausnehmung (22) ein Endbereich (14) eines ersten Verbindungselementes (2) einsetzbar ist und in einen zweiten Bereich (24) der U-förmigen Ausnehmung (22) ein Endbereich (15) eines zweiten Verbindungselementes (9) einsetzbar ist, dass zwischen die beiden Schenkel (20, 21) ein Presselement (25; 51) einsetzbar und auf die Schenkel (20, 21) Spannmittel (28) aufsetzbar sind, mit welchen das Presselement (25; 51) gegen die beiden Endbereiche (14, 15) des ersten und zweiten Verbindungselementes (2, 9) pressbar sind.

2. Wirbelsäulenimplantat nach Anspruch 1, **dadurch gekennzeichnet, dass** das Presselement (25; 51) mit Führungen (26; 52) ausgestattet ist, welche mit an den Flanken der Schenkel (20, 21) angeordneten Führungsflächen (27) zusammenwirken.

3. Wirbelsäulenimplantat nach Anspruch 1 oder 2, **dadurch gekennzeichnet, dass** die Spannmittel (28) eine Spannschraube (34; 54) umfassen, mit welcher das Presselement (25; 51) mit einer ersten Pressfläche (35) gegen den Endbereich (14) des ersten Verbindungselementes (2) und einer zweiten Pressfläche (36) gegen den Endbereich (15) des zweiten Verbindungselementes (9) pressbar ist.

4. Wirbelsäulenimplantat nach Anspruch 3, **dadurch**
**gekennzeichnet, dass** die Spannschraube (34) in eine Kappe (29) eingesetzt ist, die auf die Schenkel (20, 21) aufsetzbar und mit diesen verriegelbar sind.

5. Wirbelsäulenimplantat nach Anspruch 3, **dadurch**
**gekennzeichnet, dass** die Spannschraube (54) in ein innenseitig an den Schenkeln (20, 21) angebrachtes Gewinde (53) einschraubbar ist.

6. Wirbelsäulenimplantat nach einem der Ansprüche 1 bis 5,
**dadurch gekennzeichnet, dass** der Endbereich (14) des ersten Verbindungselementes (2) kugelförmig ausgebildet ist und dass die zwischen dem ersten Schenkel (20) und dem zweiten Schenkel (21) gebildete Grundfläche des ersten Bereichs (23) der U-förmigen Ausnehmung (22) eine konkave Kugelform aufweist, die dem kugelförmigen Endbereich (14) des ersten Verbindungselementes (2) entspricht.

7. Wirbelsäulenimplantat nach einem der Ansprüche 3 bis 6,
**dadurch gekennzeichnet, dass** die jeweiligen Pressflächen (35; 36) des Presselementes (25; 51) an die Oberflächenformen der jeweiligen Endbereiche (14; 15) der Verbindungselemente (2; 9) angepasst sind.

8. Wirbelsäulenimplantat nach einem der Ansprüche 3 bis 7,
**dadurch gekennzeichnet, dass** die zweite Pressfläche (36) des Presselementes (25; 51) und/oder die Grundfläche des zweiten Bereichs (24) der U-förmigen Ausnehmung (22) mit Strukturen (37) versehen sind.

9. Wirbelsäulenimplantat nach Anspruch 8, **dadurch**
**gekennzeichnet, dass** mindestens der Endbereich (15) des zweiten Verbindungselementes (9) mit Strukturen versehen ist, die zu den Strukturen (37) des zweiten Bereichs (24) der U-förmigen Ausnehmung (22) komplementär ausgebildet sind.

10. Wirbelsäulenimplantat nach Anspruch 8 oder 9, **dadurch gekennzeichnet, dass** die Strukturen (37) aus Rippen (38) und Rillen (39) bestehen, die im Wesentlichen quer zur Längsachse des zweiten Verbindungselementes (9) ausgerichtet sind.

11. Wirbelsäulenimplantat nach einem der Ansprüche 3 bis 10, **dadurch gekennzeichnet, dass** die Spannschraube (34; 54) drehbar mit dem Presselement (25; 51) verbunden ist.

12. Wirbelsäulenimplantat nach einem der Ansprüche 1 bis 11, **dadurch gekennzeichnet, dass** das Presselement (25) mindestens auf der Seite, welche mit dem zweiten Verbindungselement (9) zusammenwirkt, mit Abstützflächen (42) versehen ist, welche auf an den Schenkeln (20, 21) angebrachten Anschlagflächen (43) abstützbar sind.
